# EUROPEAN PATENT APPLICATION

(11) **EP 1 123 709 A1**
(43) Date of publication of application: **16.08.2001**
(21) Application number: 99949361.2
(22) Date of filing: 22.10.1999
(51) Int. Cl.: A61K 38/17, A61K 38/18, A61P 1/16

(54) **PREVENTIVES/REMEDIES FOR LIVER DISEASES**

(30) Priority: 23.10.1998 JP 30188498
(71) Applicant: REGENERON PHARMACEUTICALS, INC., Tarrytown, NY 10591-6707 (US); Sumitomo Pharmaceuticals Company, Limited, Osaka-shi, Osaka 541-8510 (JP)
(72) Inventor: NAKAGAWA, Tsutomu, Toyonaka-shi, Osaka 561-0802 (JP); KISHINO, Michiko, Suita-shi, Osaka 564-0028 (JP); TONRA, James R., North Andover, MA 01845 (US)
(74) Representative: VOSSIUS & PARTNER
(86) International application number: JP9905832
(87) International publication number: WO0024414

(57) **Abstract**

ABSTRACT

A novel medicament for prophylaxis or treatment of liver disease, comprising a neurotrophin such as BDNF as an active ingredient. Said medicament may be useful in the treatment of liver diseases such as acute and chronic hepatitis (virus-induced hepatitis, drug-induced hepatitis, autoimmune hepatitis) and hepatocirrhosis, as well as may prevent fatty liver and further may show a protecting activity of hepatocytes.

## Description

### TECHNICAL FIELD

The present invention relates to a medicament for prophylaxis or treatment of liver disease, and more particularly, to a medicament for prophylaxis or treatment of liver disease comprising a neurotrophin as an active ingredient.

### BACKGROUND ART

Liver diseases break out by various causes such as infections by hepatitis virus, etc., or undernutrition, or on the contrary, overnutrition, or over-intake of alcohol, and when the lesion progresses, such liver diseases become worse to liver cancer. The onset of many liver diseases is triggered by impairment of hepatocytes, which finally lead to morphological mutation such as hepatocyte necrosis and fatty liver. Among them, fatty liver is a pathology wherein neutral fat over-accumulates in hepatocytes, and the content of fat therein is more than 5 % of the weight of the liver. Fatty liver is classified based on causes for onset thereof into alcoholic fatty liver; obesitydiabetic fatty liver; hunger fatty liver; fatty liver caused by administration of drugs such as antibiotics, adrenocortical hormones; fatty liver caused by infection with type C hepatitis virus; or hereditary fatty liver. The pathology of fatty liver is characterized in wide-scale fatty degeneration, and a high level of biochemical parameters in blood indicating impairment of hepatocyte such as transaminase or cholinesterase. Fatty liver often accompanies hepatic fibrosis, and if the pathology lasts for a long time, it is observed that fatty liver progresses to hepatic cirrhosis or hepatic cancer at a high rate.

At present, fatty liver is basically treated with a dietary therapy and exercise therapy, or with treatment of etiological disease for fatty liver, and medication with vitamins such as vitamin E, coline, carnitin is merely supplementarily applied thereto. Besides, in addition to agents for treatment of etiological diseases such as interferon and immunosuppressors, hepatitis is often treated by administration of protectors for hepatocyte such as glycyrrhizins, ursodeoxycholic acids, glutathiones. Biochemical parameters indicating the degree of hepatocyte destruction can be lowered by administration of these medicaments, but they are merely symptomatic therapies.

Among neurotrophic factors which are secreted from target cells or neurons and glia cells·Schwann cells in the living body and show activities to maintain the survival and differentiation of neurons, neurotrophins are proteins forming a family thereof and having high structural homology with each other. The typical examples thereof are brain-derived neurotrophic factor (hereinafter, referred to as BDNF), neurotrophin 3, etc., and they are known to act as a specific ligand of trk receptors (cf. Takeshi NONOMURA, Hiroshi HATANAKA; Jikken Igaku, vol. 13, p. 376 (1995)). Neurotrophins have been studied on the clinical utility thereof as a therapeutic agent for treating a patient of neurodegenerative diseases such as ALS, etc., and further they are known as agents for treatment of diabetes or hyperlipemia (cf. Biochem. Biophysic. Res. Commu. vol. 238, p. 633 (1997)). However, an activity of treating fatty liver or an activity of protecting hepatocyte has not been reported yet.

### DISCLOSURE OF INVENTION

An object of the present invention is to provide a novel medicament for treatment or prophylaxis of liver diseases, which can prevent fat accumulation in liver (fatty liver) or hepatocyte destruction.

The present inventors have studied on pharmacological activities of BDNF, and have found that when BDNF was administered to db/db mice which are diabetic model animals (Hitoshi ISHIDA et al., Saishin Igaku, vol. 48, p. 34 (1993)), the fat accumulation in liver was well reduced, and the hepatocyte was protected, and finally accomplished the present invention with further investigations.

### BRIEF DESCRIPTION OF THE DRAWINGS

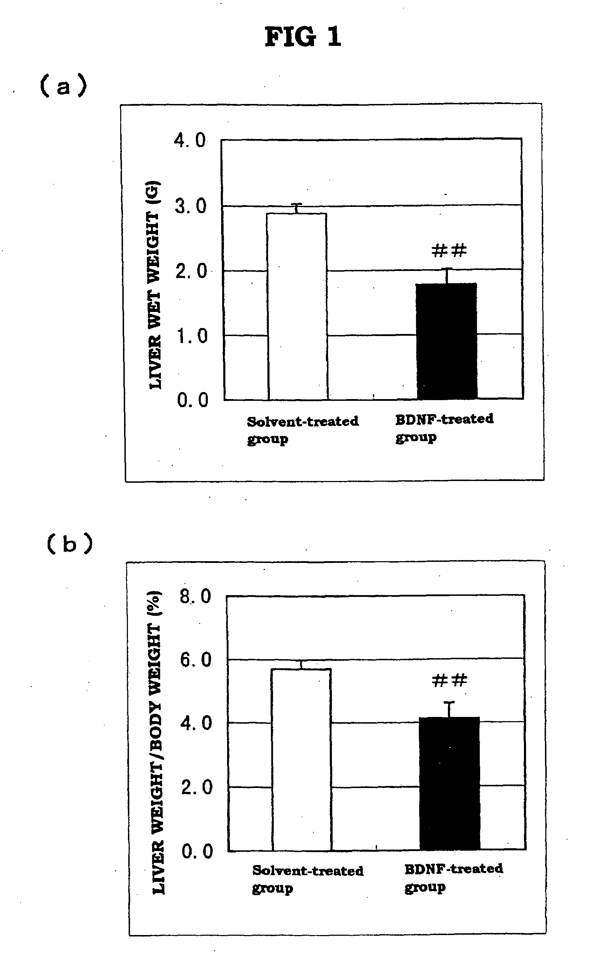

Fig. 1 shows an effect of BDNF on liver hypertrophy of db/db mice. The white column indicates the data of the solvent-treated group, and the black column indicates the data of the BDNF-treated group. The axis of ordinates shows the wet weight of liver (Fig. la), and the ratio (%) of the liver wet weight to the body weight (Fig. 1b). It is apparent that the liver hypertrophy as observed in the solvent-treated group was significantly improved in the BDNF-treated group. The data are expressed by the average values in each group (8 mice) + SD. ## indicates that there is a significant difference of P<0.01 against the solvent-treated group in Student's t test.

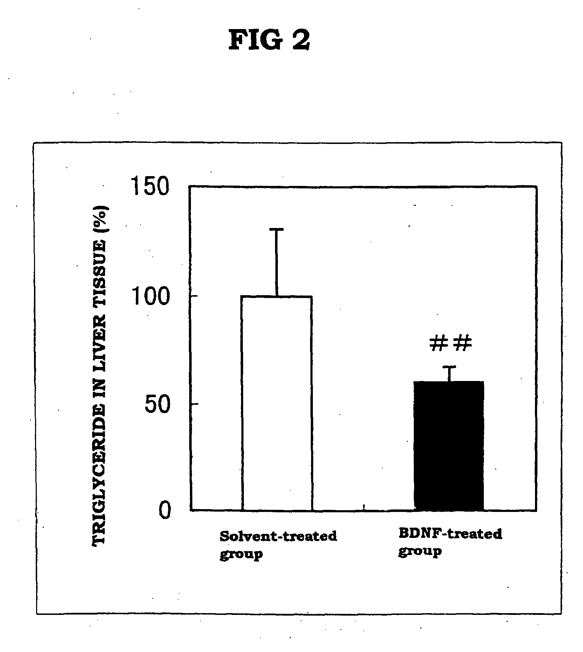

Fig. 2 shows the effect of BDNF on the fatty content in the liver tissues of db/db mice. The axis of ordinates shows relative values when the triglyceride content of the liver tissues in the solvent-treated group is regarded as 100 %. The white column indicates the data of the solvent-treated group, and the black column indicates the date of the BDNF-treated group. The data are expressed by the average value of each group (8 mice) + SD. ## indicates that there is significant difference of P<0.01 against the solvent-treated group in Student's t-test.

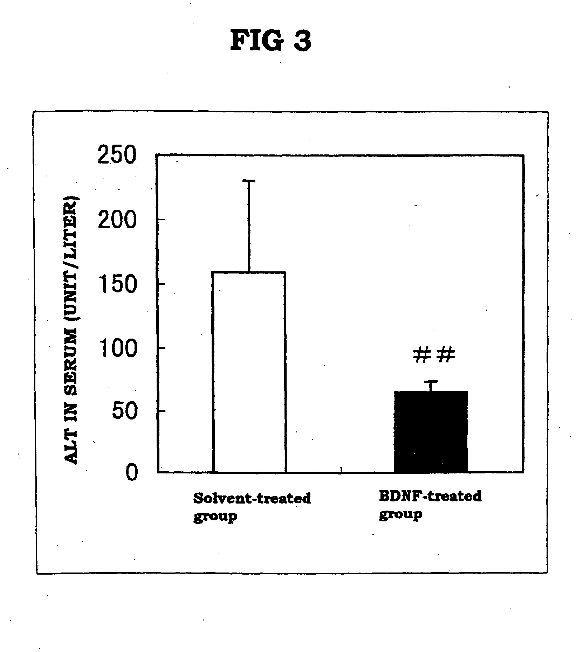

Fig. 3 shows the effect of BDNF on enzymes leaking from the liver (e.g., alaninaminotransferase (ALT)) of db/db mice. The axis of ordinates shows ALT values. The white column indicates the data of the solvent-treated group, and the black column indicates the data of the BDNF-treated group. The data are expressed by the average value of each group (8 mice) + SD. ## indicates that there is a significant difference of P<0.01 against the solvent-treated group in Student's t-test.

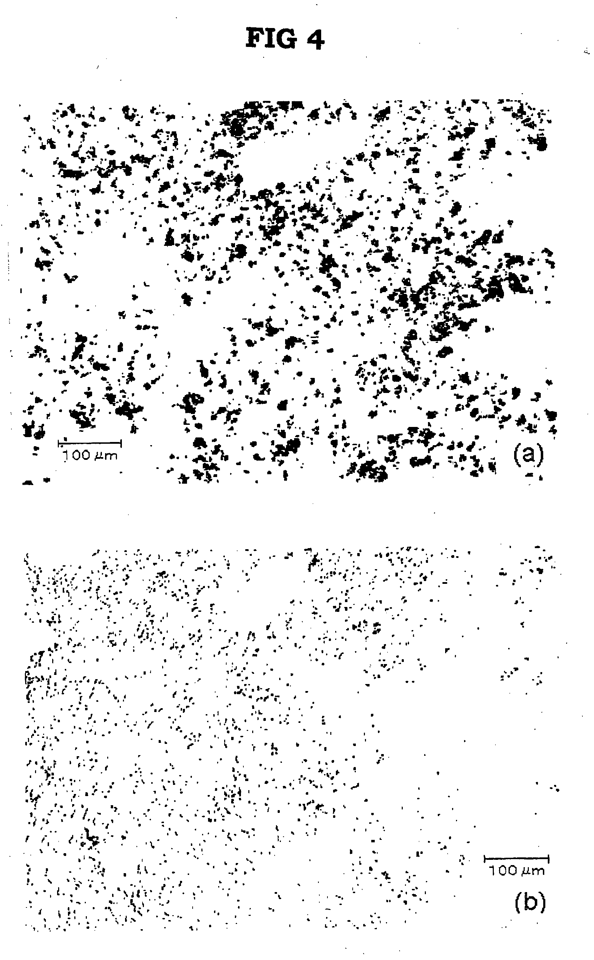

Fig. 4 shows the pictures of the liver tissues of db/db mice (the solvent-treated group: Fig. 4a; the BDNF-treated group: Fig. 4b). Oil Red 0-stained fat droplets are shown in black points.

### BEST MODE FOR CARRYING OUT THE INVENTION

That is, the present invention relates to the following medicaments:
(1) A medicament for prophylaxis or treatment of liver disease, which comprises a neurotrophin as an active ingredient;
(2) The medicament for prophylaxis or treatment of liver disease according to the above (1), wherein the neurotrophin is a brain-derived neurotrophic factor;
(3) A medicament for prophylaxis or treatment of fatty liver, which comprises a neurotrophin as an active ingredient;
(4) A medicament for prophylaxis or treatment of liver disease, which comprises an agonist of trkA, trkB and/or trkC receptors as an active ingredient;

The present invention is explained in more detail below.

The "neurotrophin" means a neurotrophic factor, which is secreted from the target cells for nerve growth, or promotes the growth, differentiation, or survival of neurons by autocrine or paracrine, and makes them form a neural circuit (synapse) in the living body. For example, a BDNF, a nerve growth factor (hereinafter, referred to as NGF), a neurotrophin-3 (hereinafter, referred to as NT-3), a neurotrophin-4 (hereinafter, referred to as NT-4), a neurotrophin-5 (hereinafter, referred to as NT-5), a neurotrophin-6 (hereinafter, referred to as NT-6), etc. are known at present. BDNF, NT-3, NT-4, NT-5, NT-6, etc. are preferable. Especially, BDNF, NT-3 and NT-4, which exhibit their biological effect via trkB or trkC receptors, are more preferable.

The "agonist of trkA, trkB and/or trkC receptors" is a general name of the products of trk gene expression being known as receptors of "neurotrophins", which activate the trkA, trkB and/or trkC receptors by binding to trkA, trkB and/or trkC, and make them work. For example, as known neurotrophins, NGF binding to trkA, BDNF and NT-4 both binding to trkB, and NT-3 binding to trkC are exemplified. Modified recombinant products of each "neurotrophin" (e.g, modified recombinant products by substitution, deletion or addition of amino acid, or by modification of sugar chain), or even peptides of small molecule, or organic compounds are also included within the concept of "agonists of trkA, trkB and/or trkC receptors" as far as such product shows an activity of binding to trkA, trkB or trkC receptors, or an activity of activating these factors, for example, phosphorization activity of tyrosine residue.

In the present specification, the "medicament for prophylaxis or treatment of liver disease" means a medicament which prevents the fat accumulation in the liver or possesses maintaining activity of hepatocyte survival, and hence, can prevent the fat accumulation in the liver and improve the liver function by administering thereof to a patient with a liver disease. Representatives of the liver disease are fatty liver, chronic hepatitis (hepatitis caused by virus-infection, drug, autoimmunity, etc.), hepatocirrhosis.

### METHODS FOR PRODUCTION AND ASSAY:

A neurotrophin used in the present medicament for prophylaxis or treatment of liver disease (e.g., NGF, BDNF, NT-3, NT-4, etc.) may be any one, for example, naturally-occurred ones, recombinant ones, as far as they show their inherent biological activities and can be used in the present invention when purified. For example, in case of a BDNF, which was isolated from porcine brain by Barde, Y.E. et al (cf. The EMBO J., vol. 5, p. 549-553 (1982)), and it's gene was analyzed after cloning whereby it was confirmed that BDNF has a primary structure consisting of 119 amino acids (cf., Leiblock, J. et al., Nature, vol. 341, p. 149 (1989)), a modified recombinant BDNF such as Met-BDNF having a methionine residue at the N-terminus, or a recombinant BDNF produced by addition, substitution, deletion or removal of a part of amino acid sequence of a natural BDNF by a conventional bio-engineering technique may be used in the present invention, as far as they are a pharmaceutical preparation having a BDNF activity.

The "BDNF activity" means an activity of maintaining survival or promoting differentiation of a dorsal root ganglia, a ganglion inferius nervi vagi, a motoneuron, a retina ganglia, a nigradopaminergic neuron, a basal forebrain cholinergic neuron, etc. These activities in the living body can be confirmed *in vitro* or *in vivo* (Japanese Patent Publication No. 5-328974, USP 5,180,820).

Various methods for preparing BDNF have been reported, and any BDNF that is prepared by any method can be used in the present invention. When a BDNF isolated from animal tissues is used in the present invention, it may be purified to such a degree that it can be used as a medicament (cf., The EMBO J., vol. 5, p. 549 (1982)). Alternatively, a BDNF can be obtained by culturing a primary culture cell or an established cell line that can produce BDNF, and isolating from the culture broth thereof (e.g., culture supernatant, cultured cells). Moreover, there may be used a recombinant BDNF which can be obtained by a conventional gene engineering technique, e.g., by inserting a gene encoding BDNF into a suitable vector, transforming a suitable host with the recombinant vector, and isolating from a culture supernatant of the resulting transformant (cf., Proc. Natl. Acad. Sci. USA, vol. 88, p. 961 (1991); Biochem. Biophys. Res. Commun., vol. 186, p. 1553 (1992)), which is suitable for production of BDNF of uniform property in a large scale. The host cells to be used in the above process is not limited, but may be any conventional host cells which have been used in a gene engineering technique, for example, *Escherichia coli*, *Bacillus subtilis*, yeasts, plant cells or animal cells. Recombinant products of other neurotrophins such as NGF, NT-3, NT-4, etc. are also reported. NGF can be produced by expressing in various host cells in a similar manner as the production of BDNF (CHO cell; Schmelzer C. H., J. Neurochemistory, vol. 59, p. 1675 (1992); *Escherichia coli*: Japanese Patent No. 2637392). NT-3 can be produced by expressing in various host cells in a similar manner as the production of BDNF. Methods for production thereof and for assay thereof are disclosed in Neuron, vol. 4, p. 767 (1990) and Japanese Patent Publication No. 5-161493 (WO 91/3659). NT-4 can be produced by expressing in various host cells in a similar manner as the production of BDNF. The method for expressing recombinant NT-4 and assay thereof are disclosed in Proc. Natl. Acad. Sci. USA, vol. 89, p. 3060 (1992), Japanese Patent Publication No. 7-509600 (WO 93/25684) and Japanese Patent Publication No. 6-501617 (WO 925254).

### RENATURATION AND PURIFICATION:

A neurotrophin produced by a recombinant technique can be re-natured or purified by a conventional method in the biochemical field. For example, treatment with denaturant and reducing agent, ion-exchange chromatography, gelfiltration, reversed phase HPLC, precipitation with ammonium sulfate, ultrafiltration, etc. and a combination thereof are used. Either polyclonal or monoclonal antibody to a neurotrophin can be produced by a conventional method. A specific antibody can be used in an antibody column or immunochemical assay such as ELISA. Reports on the renaturation or purification of neurotrophins are as follows.
(1) J. of Chromatography, vol. 632, p. 29 (1993)
(2) Biochemistry, vol. 32, p. 10812 (1993)
(3) Biochemistry, vol. 33, p. 4667 (1994)

### PREPARATION:

A pharmaceutical preparation may be an injection preparation, an oral preparation, a liquid preparation, or a lyophilized preparation, but an injection preparation for subcutaneous administration or intramuscular administration are preferable. These parenteral pharmaceutical preparations may contain a conventional stabilizer, buffering agent or carrier used in this field, for example, a protein derived from the serum such as albumin, an amino acid such as glycine, a saccharide such as mannitol, etc. can be used. Besides, a surfactant such as Tween 80 is preferably added thereto in order to prevent aggregation of an active substance, or a preservative may be added thereto for the long-term preservation. When a prolonged pharmacological effect is required, then it is possible to prepare a pharmaceutical preparation with using a conventional protein sustained-release pharmaceutical carrier (polylactic acid-polyglycolic acid co-polymer, collagen, silicone).

### USAGE:

When an active ingredient is a BDNF, the daily dosage of the medicament for prophylaxis or treatment of liver disease of the present invention is in the range of 0.5 µg - 50 mg per 1 kg of the body weight in an adult, and it can be administered intravenously, subcutaneously, or intramuscularly. The frequency of the administration may vary according to the dosage, administration route, or conditions of the patients, and is not critical, but it is possibly in the range of once a month to three times a day, and generally in the range of once a week to seven times a week, for several weeks. By the treatment with the present medicament, the hepatocytes are protected, renatured or activated, and the liver disease is improved. The medicament of the present invention can be used together with interferon or other medicaments for treatment of liver disease.

### TOXICITY:

Neurotrophins, especially BDNF, are known to be low toxic. When a BDNF was administered subcutaneously to rats and cynomolgus monkeys at a dose of 100 mg/kg and 60 mg/kg, respectively, for four weeks, no animal died. With respect to the acute toxicity, BDNF was administered to rats and cynomolgus monkeys at a dose of more than 200 mg/kg, and no animal died. Significantly serious side effects have not been reported with respect to other neurotrophins.

### EXAMPLES:

The present invention is explained by Examples.

### Example 1

Effects of BDNF on hepatic hypertrophy and fatty liver of obesity diabetic animal model (db/db mice):

### (1) Materials and Method for Experiment

Reagents: BDNF available from Regeneron Pharmaceuticals, Inc. was used. The solvent was a commercially available Dulbecco's phosphate buffered saline (D-PBS, GIBCO). The other reagents were commercially available ones of Finest Quality.

Experimental animals: Female C57BL/KsJ-db/db mice (SPF-standard) were purchased from CLEA Japan, Inc. After prebreeding, the experiment was started when the animals aged to 12-weeks old. The animals were fed in a room wherein the temperature was kept at 23±2°C (set point); the humidity was kept at 55±10 % (set point); and the illumination cycle was continuously kept as lighting-up time of 8:00-20:00 and blackout time of 20:00-8:00. The feed was solid feed (CE-2, manufactured by CLEA Japan, Inc.), and the drinking water was freely given to the animals with a feed bottle.

### (2) Preparation of BDNF solution:

A BDNF solution was prepared by diluting BDNF in D-PBS at a concentration of 3.5 mg/ml, and was administered to the animals at a dose of 35 mg/kg twice a week. The same amount of D-PBS was administered as a solvent.

### (3) Effects of BDNF on liver wet weight:

db/db Mice, which have been pre-fed, were grouped into the solvent-treated group and the BDNF-treated group (each group: 8 mice) with reference to the blood glucose level thereof as an indicator, and then BDNF or the solvent was subcutaneously administered twice a week. Three weeks thereafter, the liver was taken out from the mice, and the wet weight of the liver was measured. The results are shown in Fig. 1a. In addition, the wet weight of the liver was corrected based on the body weight of the mice, and the results thereof are shown in Fig. 1b.

As a result, the wet weight of the liver of the BDNF-treated group is significantly reduced as compared with that of the solvent-treated group. It was clear that the hepatic hypertrophy, which was often observed in the obesity diabetic animal models, was well improved in the BDNF-treated group. Besides, the inhibitory effect on hepatic hypertrophy was still statistically significantly apparent even though the wet weight of the liver was corrected based on the body weight. Thus, it was confirmed that the reduction of the wet weight of the liver is not affected by the change in the body weight.

### (4) Effects of BDNF on the lipid content in the liver tissues:

In order to study the effects of BDNF on the lipid content in the liver tissues, the content of triglyceride in the excised liver was measured (cf. Clin. Chem. vol. 36, p. 1605 (1990)). The liver was crushed, and suspended in a mixture of chloroform : methanol (2:1). To the suspension was added 0.6 % NaCl, and the mixture was stirred, and centrifuged to separate the organic layer and the aqueous. layer. The solvent was evaporated from the organic layer, and the resulting precipitate was dissolved in a phosphate buffer containing 1 % Triton X-100. The content of triglyceride in the mixture was determined with using N-Triglycerase "NISSUI" (manufactured by Nissui Pharmaceutical) (Fig. 2). The results were compared with the solvent-treated group, and the triglyceride content in the liver tissues of the BDNF-treated group was significantly reduced, and it is clear that the fatty liver is improved.

### (5) Effects of BDNF on enzymes leaking from the liver:

It has been known that the level of alanine aminotransferase (ALT) in the serum of a patient suffering from fatty liver is raised (cf., Enzyme, vol. 36, p. 266 (1986)). With knowing this fact, the blood was taken out from the db/db mice to which BDNF or a solvent had been administered for three weeks, and the ALT level in the serum was measured (Fig. 3). The results were compared with those of the solvent-treated group, and it was apparent that ALT in the BDNF-treated group was significantly low. By these results, it was confirmed that BDNF may improve the fatty liver even though an enzyme leaking from the liver was used as an indicator.

### (6) Effects of BDNF on liver tissues picture:

According to the method disclosed in Color Picture Book of Practical Pathological Tissue Cell Color Staining, p. 96 (HBJ publisher), the Oil Red O Staining was performed on the frozen graft of the liver tissue, and the fat content was observed in the liver tissues. The liver tissue picture of the solvent-treated group is shown in Fig. 4a, and that of the BDNF-treated group is shown in Fig. 4b. In Figs, the lipid droplets stained with Oil Red O are shown as black dots. As clear from Fig. 4a and Fig. 4b, the lipid droplets in the liver of the solvent-treated group are large and in abundance, while the lipid droplets in the BDNF-treated group were quite small, and it was observed that the content of fat was extremely reduced.

### Preparation 1

A BDNF-containing aqueous preparation for subcutaneous administration, which is a representative of the present medicaments for prophylaxis or treatment of liver disease, is prepared as follows.

(1) To a recombinant BDNF (20 mg, obtained from Regeneron Pharmaceuticals, Inc.) are added mannitol (10 mg), nonionic surfactant; Polysolvate 80 (0.1 mg), and the mixture is dissolved in a phosphate buffer (1 ml, pH 7.0, 10 mM).

### INDUSTRIAL APPLICABILITY

The medicament for prophylaxis or treatment of liver disease of the present invention may improve various liver diseases by activities of preventing fatty liver and of protecting hepatocytes.

## Claims

1. A medicament for prophylaxis or treatment of liver disease, which comprises a neurotrophin as an active ingredient.

2. The medicament for prophylaxis or treatment of liver disease according to claim 1, wherein the neurotrophin is a brain-derived neurotrophic factor.

3. A medicament for prophylaxis or treatment of fatty liver, which comprises a neurotrophin as an active ingredient.

4. A medicament for prophylaxis or treatment of liver disease, which comprises a ligand for trkA, trkB or trkC receptor as an active ingredient.

5. A use of neurotrophin in the preparation of a medicament for prophylaxis or treatment of liver disease.

6. The use according to claim 5, wherein the neurotrophin is a brain-derived neurotrophic factor.

7. A method for prophylaxis or treatment of liver disease, which comprises administering an effective amount of a neurotrophin to a patient being suffering from a liver disease.

8. The method according to claim 7, wherein a neurotrophin is administered intravenously, subcutaneously or intramuscularly.

9. The method according to claim 7 or claim 8, wherein the neurotrophin is a brain-derived neurotrophic factor.
